# EUROPEAN PATENT SPECIFICATION

(11) **EP 0 583 837 B1**
(45) Date of publication and mention of the grant of the patent: **19.11.1997**
(21) Application number: 93202396.3
(22) Date of filing: 16.08.1993
(51) Int. Cl.: B01J 37/00, C07C 1/04, B01J 23/40, B01J 23/74

(54) **Process for the preparation of a catalyst for a catalyst precursor**
Verfahren zur Herstellung von einem Katalysator oder von einem Katalysatorvorläufer
Procédé de préparation d'un catalyseur ou d'un précurseur de catalyseur

(30) Priority: 18.08.1992 EP 92202538
(43) Date of publication of application: 23.02.1994
(73) Proprietor: SHELL INTERNATIONALE RESEARCH MAATSCHAPPIJ B.V., 2596 HR Den Haag (NL)
(72) Inventor: Rek, Paulus Johannes Maria, NL-1031 CM Amsterdam (NL); Blankenstein, Paul, NL-1031 CM Amsterdam (NL)

(56) References cited:
- EP-A- 0 313 163
- EP-A- 0 455 307
- GB-A- 2 098 083
- GB-A- 2 118 062

## Description

The present invention relates to a process for the preparation of a catalyst or catalyst precursor, in particular to the preparation of a catalyst or a catalyst precursor by a combination of mulling and extrusion.

It is known to prepare a catalyst or catalyst precursor by a process comprising first mulling a refractory oxide or precursor thereof with a solvent and, thereafter, extruding the resulting mixture through a suitably shaped orifice. Further, it is known that, to facilitate the extrusion of the mixture produced in the mulling stage and improve the properties of the final extrudates, it is advantageous to peptise the refractory oxide or precursor thereof prior to extrusion.

Thus, in European patent application publication No. 0 167 324 (EP-A-0 167 324) there is disclosed a method for extruding silica-rich solids comprising mixing the silica-rich solids with water and an alkali metal compound, mulling the mixture, extruding the mixture and drying the extrudates so-formed. The presence of the alkali metal compound gives rise to the basic conditions necessary to peptise the silica prior to extrusion. However, in many applications, the presence of alkali metal ions in the final extrudates can adversely affect the catalytic behaviour of the eventual catalyst. The alkali metal ions may be removed from the extrudates of EP-A-0 167 324 by soaking in a solution of ammonium nitrate and nitric acid. However, the need for such a step in the preparation of a commercial catalyst may be undesirable. Accordingly, there is a need for a process for preparing refractory oxide or silica extrudates which is not reliant on the presence of alkali metals in the extrusion mixture.

European patent application publication No. 0 309 048 (EP-A-0 309 048) discloses a process for the preparation of a shapable dough comprising mixing and kneading a particulate silica with water and ammonia or an ammonia-releasing agent and extruding the resulting mixture. European patent application publication No. 0 313 163 (EP-A-0 313 163) discloses a similar process for the preparation of silica-alumina extrudates, in which the silica-alumina is peptised using an alkanolamine or ammonia.

Further, European patent application publication No. 0 455 307 (EP-A-0 455 307) discloses a process for the preparation of alumina-based extrudates comprising preparing an extrudable mixture comprising an alumina precursor, a cobalt, iron or nickel source and a solvent, extruding the mixture and drying the resulting extrudates. It is disclosed in EP-A-0 455 307 that the mixture to be extruded may comprise an agent to peptise the alumina, for example weak acids, such as formic acid, acetic acid and propionic acid.

European patent application publication No. 0 389 041 (EP-A-0 389 041) discloses a process for the preparation of titania-based extrudates comprising preparing a shapable dough by mixing and kneading a particulate titania with water and an alkanolamine, ammonia or an ammonia-releasing compound and, thereafter, extruding the dough.

Recently, a process has been disclosed for the preparation of a catalyst or catalyst precursor in which a mixture of a refractory oxide or precursor thereof is comulled with a solvent and a source for a catalytically active component, which source is also active as a peptising agent. Thus, European patent application publication No. 0 428 223 (EP-A-0 428 223) discloses a process for the preparation of extrudates comprising mulling a mixture of finely divided silica, a water soluble compound derived from a metal selected from Group IVB of the Periodic Table and water. It is disclosed in EP-A-0 428 223 that use of a basic compound of a metal of Group IVB reduces or removes the need for an additional basic compound to be included in the mixture in order to fully peptise the silica prior to extrusion. A similar use of a compound to act both as a source for a catalytically active component in the final catalyst and as a peptising agent is described in the specifications of United Kingdom patent applications Nos. 9108656.1, 9108663.7 and 9108657.9 (corresponding to European patent applications publication Nos. 0 510 772, 0 510 771 and 0 510 770 respectively).

When preparing a catalyst or catalyst precursor, the inclusion of an acidic or a basic compound in the mixture prior to extrusion leads to a lowering or raising respectively of the pH of the mixture. It has been found that a mixture having a pH significantly lower or higher than the neutral pH is more difficult to extrude than a mixture having a pH in the range of about 6 to 8. In the Examples of EP-A-0 428 223, acetic acid is added to the mixture being mulled to reduce the pH of the mixture to an acceptable level prior to extrusion.

United Kingdom patent application publication Nos. 2 098 083 and 2 118 062 describe catalyst preparation processes in which metal salts may be added to solutions of peptising agents or neutralising agents.

Surprisingly, a process has been found which allows the optimum preparation of a catalyst or catalyst precursor by means of the mulling and extrusion techniques hereinbefore described, but which allows the catalyst or catalyst precursor to be prepared using a minimum number of ingredients in the mixture being mulled. The process arises from the finding that the neutralising agent and the peptising agent employed during the mulling and extrusion procedure may be a source of a catalytically active component.

Accordingly, the present invention provides a process for the preparation of a catalyst or catalyst precursor, which process comprises mulling a mixture comprising a refractory oxide or refractory oxide precursor, a solvent and a peptising agent; adding to the mixture a neutralising agent, extruding the resulting mixture, and drying the resulting extrudate, wherein the neutralising agent and the peptising agent are sources of at least one catalytically active component, and are added as salts or basic complexes of said at least one catalytically active component.

The process of the present invention advantageously provides a simple process for the preparation of a catalyst or catalyst precursor. The process allows the conditions during the mulling and extrusion stages to be controlled, thereby resulting in products having optimum properties, using a minimum number and amount of compounds. In this way, the need for introducing into the mixture agents which are not required in terms of the final product composition and which may even adversely affect the properties of the final catalyst is reduced and, in many cases removed.

The refractory oxide for inclusion in the mixture may be any suitable refractory oxide, for example alumina, silica, titania, zirconia, silica-alumina and mixtures thereof. Silica and alumina are most suitable refractory oxides. The aforementioned refractory oxides are available commercially and are well known as materials for use in the preparation of catalysts and catalyst precursors.

As an alternative or in addition to the refractory oxide, the mixture being mulled may comprise a refractory oxide precursor. A refractory oxide may be prepared by heating the corresponding hydroxide. As the heating progresses, the hydroxide is converted via a number of intermediate forms and the successive loss of a number of water molecules into the refractory oxide. For the purposes of this specification, the term "refractory oxide precursor" is to be taken as a reference to the corresponding hydroxide or any of the aforementioned intermediate forms.

The solvent may be any of the suitable solvents known in the art, for example water; alcohols, such as methanol, ethanol and propanol; ketones, such as acetone; aldehydes, such as propanal; and aromatic solvents, such as toluene. A most convenient and preferred solvent is water.

The mixture to be extruded should have a solids content in the range of from 20 to 60% by weight, more preferably from 30 to 50% by weight.

In the process of the present invention, the mixture to be mulled comprises, in addition to one or more refractory oxides or precursors thereof, a peptising agent. The peptising agent may be either an acidic compound or a basic compound, depending upon the particular refractory oxide present in the mixture. The addition of the acidic or basic peptising agent lowers or raises respectively the pH of the mixture being mulled. Once the required degree of peptising of the refractory oxide has been achieved, a neutralising agent is added to the mixture to neutralise the peptising agent and return the pH to a value in the range of about 6 to 8.5. The neutralising agent will be either a basic compound or an acidic compound, depending upon the particular refractory oxide present in the mixture.

In the process of this invention, the neutralising agent and the peptising agent are sources of at least one catalytically active component. Thus, the peptising agent and neutralising agent may be sources of the same or different catalytically active components. Further, one or both of the peptising agent and the neutralising agent may, if desired, comprise a mixture of compounds and, thus, act as a sources of a plurality of catalytically active components in the final catalyst.

For the purposes of this specification, the term "catalytically active component" is a reference to elements active as catalysts with respect to one or more reactions, as well as elements active as co-catalysts or promoters. Accordingly, the neutralising agent and the peptising agent may be sources of one or more elements selected from Groups Ib, IIB, IIIB, IVB, VB, VIB, VIIB, VIII of the Periodic Table of Elements, or the Lanthanides and Actinides. Preferably, the said agents are sources of one or more elements in Group VIII of the Periodic Table, in particular elements selected from iron, ruthenium, cobalt, rhenium, nickel, palladium and platinum, especially cobalt, iron and nickel, or elements from Group IVB of the Periodic Table, in particular titanium and/or zirconium.

As mentioned hereinbefore, the peptising agent may be either an acidic compound or a basic compound, depending upon the nature of the refractory oxide. The particular type of peptising agent required for a given refractory oxide is well known in the art. For example, alumina requires an acidic compound for peptising to occur.

Suitable acidic peptising agents include both organic and inorganic acid salts of the catalytically active components mentioned hereinbefore. Examples of suitable compounds include acetates, propanoates, butanoates, carbonates, nitrates, oxalates, citrates and phosphates. Salts of organic acids are convenient and preferred acidic peptising agents, with acetates being especially preferred.

In contrast, suitable peptising agents for silica, titania and zirconia are basic compounds. Suitable basic compounds include both organic and inorganic basic salts of the catalytically active components mentioned hereinbefore. Examples of suitable compounds include carbonates, phosphates and basic complexes. Ammonium carbonate complexes are particularly suitable and preferred compounds for use as basic peptising agents.

The amount of peptising agent included in the mixture should be sufficient to peptise the refractory oxide and/or refractory oxide precursor present in the mixture. The amount present may be readily determined by measuring the pH of the mixture. If a basic peptising agent is being used, the pH of the mixture should be in the range of from 8 to 11.5, more preferably from 9 to 11. If an acidic peptising agent is being used, the pH should be in the range of from 1 to 6, more preferably from 4 to 6.

Once the refractory oxide and/or refractory oxide precursor has been peptised to the required degree, a neutralising agent is added to the mixture to stop the peptising reactions. As mentioned hereinbefore, the neutralising agent is a basic compound or an acidic compound, depending upon the nature of the refractory oxide present in the mixture. The neutralising agent is a source of a catalytically active component. Suitable basic and acidic compounds for use as neutralising agents have been discussed hereinbefore with respect to the peptising agents. Ammonium carbonate complexes are particularly suitable for use as basic neutralising agents. Acetates are particularly suitable acidic neutralising agents.

To facilitate extrusion of the final mixture and yield extrudates of high quality, sufficient neutralising agent should be added to the mixture to return the pH to a value in the range of from 6 to 8.5.

The process of the present invention provides the advantage that no additional neutralising agents are required.

To improve the flow properties of the mixture, it is preferred to include one or more flow improving agents and/or extrusion aids in the mixture prior to extrusion. Suitable additives for inclusion in the mixture include fatty amines, quaternary ammonium compounds, polyvinyl pyridine, sulphoxonium, sulphonium, phosphonium and iodonium compounds, alkylated aromatic compounds, acyclic mono-carboxylic acids, fatty acids, sulphonated aromatic compounds, alcohol sulphates, ether alcohol sulphates, sulphated fats and oils, phosphonic acid salts, polyoxyethylene alkylphenols, polyoxyethylene alcohols, polyoxyethylene alkylamines, polyoxyethylene alkylamides, polyacrylamides, polyols and acetylenic glycols. Preferred additives are sold under the trademarks Nalco and Superfloc.

The flow improving agents/extrusion aids are preferably present in the mixture in a total amount in the range of from 1 to 20% by weight, more preferably from 2 to 10% by weight, on the basis of the total weight of the mixture.

It has been found advantageous to prepare and mull the mixture in the following manner. The refractory oxide and/or refractory oxide precursor, solvent and the peptising agent are combined and the resulting mixture mulled. Once the refractory oxide and/or precursor thereof have been sufficiently peptised, the neutralizing agent is added and the resulting mixture mulled for a further period of time. Thereafter, any flow improving agents and/or extrusion aids may be added and the mixture mulled for a final period of time before extrusion of the mixture.

Typically, the mixture is mulled for a total period of from 10 to 120 minutes, preferably from 15 to 90 minutes. During the mulling process, energy is input into the mixture by the mulling apparatus. The rate of energy input into the mixture is typically in the range of from 0.05 to 50 Wh/min/kg, preferably from 0.5 to 10 Wh/min/kg. The mulling process may be carried out at a broad range of temperature, preferably from 15 to 50 °C. As a result of the energy input into the mixture during the mulling process, there will be a rise in temperature of the mixture during the mulling. The mulling process is conveniently carried out at ambient pressure. Any suitable, commercially available mulling machine may be employed.

Once the mulling process has been completed, the resulting mixture is extruded. Extrusion may be effected using any conventional, commercially available extruder. In particular, a screw-type extruder may be used to force the mixture through one or more orifices in a suitable dieplate to yield extrudates having the desired form. The strands formed upon extrusion may be cut or broken to the desired length.

The extrudates may have any of the forms known in the art, such as cylinders, for example hollow cylinders, a multilobed or twisted multilobed form. The process of the present invention has been found to be particularly suitable for forming trilobe extrudates. The extrudates may have any suitable nominal diameter, typically in the range of from 0.5 to 5 mm, preferably from 1 to 3 mm, for many applications.

After extrusion, the extrudates are dried. Drying may be effected at an elevated temperature, preferably up to 800 °C, more preferably up to 300 °C. The period for drying is typically up to 5 hours, more preferably from 30 minutes to 3 hours.

Preferably, the extrudates are calcined after drying. Calcination is effected at an elevated temperature, preferably up to 1000 °C, more preferably from 200 to 1000 °C, most preferably from 300 to 800 °C. Calcination of the extrudates is typically effected for a period of up to 5 hours, preferably from 30 minutes to 4 hours. The calcination may be effected, for example, by heating the extrudates in air, or by means of direct heating using the hot exhaust gases of a flame to contact the extrudates.

The extrudates prepared by the process of the present invention may be employed directly as catalyst particles, after the application of any necessary activation procedures. Alternatively, one or more catalytically active components may be deposited on the extrudates, which catalytically active components may be the same or different to those present in the finished extrudates. Suitable techniques for depositing catalytically active component are known in the art. A particularly suitable technique is impregnation.

The products of the process of the present invention find use in any process in which a catalyst comprising a catalytically active component, as hereinbefore defined, and a refractory oxide carrier is required. In particular, when the catalytically active component is active as a Fischer-Tropsch catalyst, the products may be employed in the synthesis of hydrocarbons from a mixture of carbon monoxide and hydrogen, the so-called Fischer-Tropsch synthesis. Typically, the hydrocarbon synthesis reaction is effected at a temperature in the range of from 125 to 350 °C, more preferably from 175 to 250 °C. The reaction pressure is typically in the range of from 5 to 100 bar, more preferably from 10 to 50 bar. The hydrogen/carbon monoxide ratio in the feed is typically greater than 1.5, preferably from 1.75 to 2.25, with unconverted carbon monoxide and hydrogen being recycled to contact the catalyst for a second time.

The process of the present invention is further described in the following illustrative examples. In the examples, the values for the loss on ignition are quoted on the basis of the water lost upon heating the sample to a temperature in the range of from 550 to 600 °C.

### Example 1

Silica powder (precipitated silica, 87g; 75g SiO₂ on a dry basis), ammonium zirconium carbonate (Bacote, 45g; 8.8g equivalent ZrO₂) and water (84.3g) were combined and the resulting mixture mulled for a period of 10 minutes. Zirconium acetate (15.7g; equivalent 3.4g ZrO₂) and water (10g) were added and the resulting mixture mulled for a further 10 minutes. Water (11g) was added and the mixture mulled for a further 15 minutes. Finally, polyelectrolyte (Nalco, 3.5g as a 4% aqueous solution) and water (4.5g) were added and the mixture mulled for a final period of 5 minutes minutes, after which the mixture had a loss on ignition of 65.5 % and a pH of 8.2. The resulting mixture was extruded using a 1" Bonnot extruder having a 2.2 mm Delrin trilobe dieplate insert to yield trilobe extrudates. The extrudates were dried at a temperature of 120 °C and thereafter calcined in air at a temperature of 800 °C for a period of 2 hours.

The final extrudates comprised 12 parts by weight of zirconium (as Zr) per 100 parts by weight of silica.

### Example 2

HMPA (High micropore alumina, 131g; 100g alumina on a dry basis), zirconium acetate (49.1g; 10.8g equivalent ZrO₂) and water (70g) were combined and the mixture mulled for a period of 10 minutes. Ammonium zirconium carbonate (Bacote, 27.8g; equivalent 5.4g ZrO₂) and water (7.1g) were added and the resulting mixture mulled for a further 5 minutes. Extrusion aid (Superfloc, 1.1g as a 1% aqueous solution) was added. Mulling was continued for a period of 15 minutes, with water (4g) being added after each successive period of 5 minutes. Mulling was again continued for 5 minutes, after which polyelectrolyte (Nalco, 2.2g as a 2% aqueous solution) was added to yield a resulting mixture having a loss on ignition of 59.3% and a pH of 6.8. The final mixture was extruded using a 1" Bonnot extruder with a 1.7 mm trilobe dieplate insert to yield trilobe extrudates. The resulting extrudates were dried at a temperature of 120 °C and thereafter calcined at a temperature of 630 °C for a period of 2 hours.

The final extrudates comprised 12 parts by weight of zirconium (as Zr) per 100 parts by weight of alumina.

## Claims

1. A process for the preparation of a catalyst or catalyst precursor, which process comprises mulling a mixture comprising a refractory oxide or refractory oxide precursor, a solvent and a peptising agent; adding to the mixture a neutralising agent, extruding the resulting mixture, and drying the resulting extrudate, wherein the neutralising agent and the peptising agent are sources of at least one catalytically active component and are added as salts or basic complexes of said at least one catalytically active component.

2. A process according to claim 1, characterised in that the mixture comprises a refractory oxide or a precursor of a refractory oxide selected from alumina, silica, titania, zirconia, silica-alumina, preferably alumina or silica.

3. A process according to claim 1 or 2, characterised in that the solvent is water, an alcohol, an aldehyde, a ketone or an aromatic solvent, preferably water.

4. A process according to any preceding claim, characterised in that the mixture has a solids content of from 20 to 60 % by weight, preferably 30 to 50 % by weight, prior to extrusion.

5. A process according to any preceding claim, characterised in that the neutralising agent and the peptising agent are sources for a catalytically component selected from elements of Groups Ib, IIB, IIIB, IVB, VB, VIB, VIIB or VIII of the Periodic Table of Elements, or the Lanthanides and Actinides, preferably an element in Group VIII of the Periodic Table, in particular an element selected from iron, ruthenium, cobalt, rhenium, nickel, palladium or platinum, especially cobalt, iron or nickel, or an element from Group IVB of the Periodic Table, in particular titanium or zirconium.

6. A process according to any preceding claim, characterised in that said salt is an organic acid salt, preferably an acetate.

7. A process according to any preceding claim, characterised in that the basic complex is an ammonium carbonate complex.

8. A process according to any preceding claim, characterised in that after the addition of the peptising agent the mixture has a pH of from 8 to 11, preferably from 9 to 11, in the case of an basic peptising agent, and a pH of from 1 to 6, preferably from 4 to 6, in the case of an acidic peptising agent.

9. A process according to any preceding claim, characterised in that after addition of the neutralising agent the mixture has a pH in the range of from 6 to 8.5.

10. A process according to any preceding claim, characterised in that a flow improver and/or extrusion aid is added to the mixture prior to extrusion.

11. A process according to any preceding claim, characterised in that the mixture is mulled for a total period of from 10 to 120 minutes, preferably from 15 to 90 minutes.

## Patentansprüche

1. Verfahren zur Herstellung eines Katalysators oder Katalysatorvorläufers, welches Verfahren ein Mahlen eines Gemisches, das ein feuerfestes Oxid oder einen Vorläufer eines feuerfesten Oxids, ein Lösungsmittel und ein Peptisierungsmittel umfaßt; ein Zusetzen eines Neutralisierungsmittels zu dem Gemisch, ein Extrudieren des erhaltenen Gemisches und ein Trocknen des erhaltenen Extrudats umfaßt, worin das Neutralisierungsmittel und das Peptisierungsmittel Quellen wenigstens einer katalytisch aktiven Komponente sind und als Salze oder basische Komplexe dieser wenigstens einen katalytisch aktiven Komponente zugesetzt werden.

2. Verfahren nach Anspruch 1, dadurch gekennzeichnet, daß das Gemisch ein feuerfestes Oxid oder einen Vorläufer eines feuerfesten Oxids, ausgewählt unter Aluminumoxid, Siliciumdioxid, Titandioxid, Zirkonoxid, Siliciumdioxid-Aluminiumoxid, vorzugsweise Aluminiumoxid oder Siliciumdioxid, umfaßt.

3. Verfahren nach Anspruch 1 oder 2, dadurch gekennzeichnet, daß das Lösungsmittel Wasser, ein Alkohol, ein Aldehyd, ein Keton oder ein aromatisches Lösungsmittel, vorzugsweise Wasser, ist.

4. Verfahren nach einem der vorstehenden Ansprüche, dadurch gekennzeichnet, daß das Gemisch einen Feststoffgehalt von 20 bis 60 Gew.-%, vorzugsweise 30 bis 50 Gew.-% vor der Extrusion aufweist.

5. Verfahren nach einem der vorstehenden Ansprüche, dadurch gekennzeichnet, daß das Neutralisierungsmittel und das Peptisierungsmittel Quellen für eine katalytische Komponente, ausgewählt unter Elementen der Gruppen Ib, IIB, IIIB, IVB, VB, VIB, VIIB, oder VIII des Periodensystems der Elemente, oder der Lanthaniden und Actiniden, vorzugsweise ein Element in Gruppe VIII des Periodensystems, insbesondere ein unter Eisen, Ruthenium, Kobalt, Rhenium, Nickel, Palladium oder Platin ausgewähltes Element, insbesondere Kobalt, Eisen oder Nickel, oder ein Element aus Gruppe IVB des Periodensystems, insbesondere Titan oder Zirkon, sind.

6. Verfahren nach einem der vorstehenden Ansprüche, dadurch gekennzeichnet, daß das Salz ein Salz einer organischen Säure ist, vorzugsweise ein Acetat.

7. Verfahren nach einem der vorstehenden Ansprüche, dadurch gekennzeichnet, daß der basische Komplex ein Ammoniumkarbonatkomplex ist.

8. Verfahren nach einem der vorstehenden Ansprüche, dadurch gekennzeichnet, daß nach der Zugabe des Peptisierungsmittel das Gemisch einen pH-Wert von 8 bis 11, vorzugsweise von 9 bis 11 im Falle eines basischen Peptisierungsmittels und einen pH-Wert von 1 bis 6, vorzugsweise von 4 bis 6 im Falle eines sauren Peptisierungsmittels aufweist.

9. Verfahren nach einem der vorstehenden Ansprüche, dadurch gekennzeichnet, daß nach der Zugabe des Neutralisierungsmittels das Gemisch einen pH-Wert im Bereich von 6 bis 8,5 aufweist.

10. Verfahren nach einem der vorstehenden Ansprüche, dadurch gekennzeichnet, daß vor dem Extrudieren dem Gemisch ein Fließverbesserungsmittel und/oder ein Extrusionshilfsmittel zugesetzt wird.

11. Verfahren nach einem der vorstehenden Ansprüche, dadurch gekennzeichnet, daß das Gemisch während einer Gesamtzeit von 10 bis 120 Minuten, vorzugsweise von 15 bis 90 Minuten gemahlen wird.

## Revendications

1. Procédé pour la préparation d'un catalyseur ou précurseur de catalyseur, lequel procédé comprend le malaxage d'un mélange comprenant un oxyde réfractaire ou précurseur d'oxyde réfractaire, un solvant et un agent peptisant, l'addition au mélange d'un agent neutralisant, l'extrusion du mélange résultant et le séchage de l'extrudat résultant, dans lequel l'agent neutralisant et l'agent peptisant sont des sources d'au moins un composant catalytiquement actif et sont ajoutés sous la forme de sels ou complexes basiques d'au moins ce composant catalytiquement actif.

2. Procédé suivant la revendication 1, caractérisé en ce que le mélange comprend un oxyde réfractaire ou un précurseur d'un oxyde réfractaire choisi parmi l'alumine, la silice, l'oxyde de titane, la zircone, la silice-alumine, avantageusement l'alumine ou la silice.

3. Procédé suivant l'une ou l'autre des revendications 1 et 2, caractérisé en ce que le solvant est de l'eau, un alcool, un aldéhyde, une cétone ou un solvant aromatique, avantageusement de l'eau.

4. Procédé suivant l'une quelconque des revendications précédentes, caractérisé en ce que le mélange a une teneur en matières solides de 20 à 60 % en poids, avantageusement de 30 à 50 % en poids, avant extrusion.

5. Procédé suivant l'une quelconque des revendications précédentes, caractérisé en ce que l'agent neutralisant et l'agent peptisant sont des sources d'un composant catalytiquement actif choisi parmi les éléments des Groupes IB, IIB, IIIB, IVB, VB, VIB, VIIB et VIII du Tableau Périodique des Eléments ou des lanthanides et actinides, avantageusement un élément du Groupe VIII du Tableau Périodique, en particulier un élément choisi parmi le fer, le ruthénium, le cobalt, le rhénium, le nickel, le palladium et le platine, en particulier le cobalt, le fer ou le nickel, ou un élément du Groupe IVB du Tableau Périodique, en particulier le titane ou le zirconium.

6. Procédé suivant l'une quelconque des revendications précédentes, caractérisé en ce que le sel précité est un sel d'acide organique, avantageusement un acétate.

7. Procédé suivant l'une quelconque des revendications précédentes, caractérisé en ce que le complexe basique est un complexe de carbonate d'ammonium.

8. Procédé suivant l'une quelconque des revendications précédentes, caractérisé en ce qu'après l'addition de l'agent peptisant le mélange a un pH de 8 à 11, avantageusement de 9 à 11, dans le cas d'un agent peptisant basique, et un pH de 1 à 6, avantageusement de 4 à 6, dans le cas d'un agent peptisant acide.

9. Procédé suivant l'une quelconque des revendications précédentes, caractérisé en ce qu'après l'addition de l'agent neutralisant le mélange a un pH dans la gamme de 6 à 8,5.

10. Procédé suivant l'une quelconque des revendications précédentes, caractérisé en ce qu'un agent améliorant l'écoulement et/ou adjuvant d'extrusion est ajouté au mélange avant l'extrusion.

11. Procédé suivant l'une quelconque des revendications précédentes, caractérisé en ce que le mélange est malaxé pendant une période totale de 10 à 120 minutes, avantageusement de 15 à 90 minutes.
